# EUROPÄISCHE PATENTSCHRIFT

(11) **EP 2 023 977 B1**
(45) Veröffentlichungstag und Bekanntmachung des Hinweises auf die Patenterteilung: **13.10.2010**
(21) Anmeldenummer: 07701875.2
(22) Anmeldetag: 13.02.2007
(51) Int. Cl.: A61M 5/158, A61M 25/06

(54) **ANORDNUNG ZUM ZUFÜHREN EINER FLÜSSIGKEIT IN DEN KÖRPER EINES PATIENTEN**
ARRANGEMENT FOR INTRODUCING A LIQUID INTO THE BODY OF A PATIENT
SYSTÈME D'INTRODUCTION DE LIQUIDE DANS L'ORGANISME D'UN PATIENT

(30) Priorität: 02.06.2006 CH 888062006
(43) Veröffentlichungstag der Anmeldung: 18.02.2009
(73) Patentinhaber: F.HOFFMANN-LA ROCHE AG, 4070 Basel (CH); Roche Diagnostics GmbH, 68305 Mannheim (DE)
(72) Erfinder: WYSS, Martin, CH-3510 Konolfingen (CH); SCHEURER, Simon, CH-3006 Bern (CH); AESCHLIMANN, Reto, CH-3426 Aefligen (CH); THALMANN, Christian, CH-6365 Kehrsiten (CH)
(74) Vertreter: Schalch, Rainer
(86) Internationale Anmeldenummer: PCT/CH2007/000075
(87) Internationale Veröffentlichungsnummer: WO 2007/140632

(56) Entgegenhaltungen:
- EP-A1- 1 616 594
- WO-A-02/053220
- DE-U1-202004 017 861

## Beschreibung

### TECHNISCEMS GEBIET

Die vorliegende Erfindung betrifft eine Anordnung zum insbesondere subkutanen Zuführen einer Flüssigkeit in den Körper eines Patienten oder zur Abführung einer Flüssigkeit aus dem Körper eines Patienten.

### STAND DER TECHNIK

Bei Patienten, welche einen regelmässigen Bedarf an einem subkutan verabreichbaren Medikament haben, wie z.B. bei bestimmten Gruppen von Schmerzpatienten oder bei Patienten mit Diabetes Typ I und Typ II, kann es sinnvoll sein, dem Körper die erforderliche Medikamentenmenge in flüssiger Form mittels einer über einen längeren Zeitraum im subkutanen Gewebe angeordneten Kanüle zuzuführen. Hierzu wird auf der Haut des Patienten eine je nach Ausführung als "Infusionsset" oder "Port" bezeichnete Anordnung mit einer Kanüle aus einem elastischen Material (Softkanüle) befestigt, derart, dass die Kanüle durch die Haut bis in das subkutane Gewebe eindringt. Um die Kanüle durch die Haut in das subkutane Gewebe einstechen zu können, wird sie während des Applizierens von einer sich innerhalb ihres Kanülenkanals erstreckenden Führungsnadel gestützt, welche anschliessend aus der Kanüle entfernt wird, um den Kanülenkanal für die Medikamentenförderung freizugeben. Sodann wird die Kanüle, im Falle eines Infusiossets, durch Anschliessen eines von einer Medikamentendosiervorrichtung gespeisten Zuführungskatheters an die Anordnung oder, im Falle eines Ports, durch ein in gewissen Zeitabständen mit einer Spritze über ein Septum aufzufüllendes Medikamentenreservoir der Anordnung mit der erforderlichen Menge des flüssigen Medikaments gespeist.

Um Infektionen zu vermeiden, muss das Infusionsset bzw. der Port in regelmässigen Zeitintervallen gewechselt werden, z.B. alle drei Tage, was bei der nicht stationären Behandlung, z.B. bei Diabetikern, oftmals durch den Patienten selbst erfolgt. Entsprechend wichtig ist es, dass solche Infusionssets bzw. Ports kostengünstig sind und einfach und sicher zu applizieren bzw. zu entfernen sind, und dass Bedienungsfehler durch deren konstruktive Ausgestaltung möglichst von vornherein ausgeschlossen werden.

US 6, 017, 328 offenbart verschiedene Infusionssets, bei denen die Führungsnadel nach dem Einstechen der Kanüle ins Gewebe manuell an einem Griffteil oder mittels einer federunterstützten Rückziehvorrichtung in axialer Richtung vom Infusionsset entfernt wird. Hierbei besteht jedoch eine nicht unerhebliche Gefahr, dass die Kanüle unbemerkt ganz oder teilweise aus der Einstichstelle herausgezogen wird und anschliessend eine Zuführung von flüssigem Medikament in das subkutane Gewebe nicht oder nur unzureichend möglich ist. Auch besteht, insbesondere bei erstgenannter Variante, die Gefahr, dass sich der Anwender mit der ungeschützten Führungsnadel verletzt.

Die Dokumente EP 1 616 594 A1, DE 20 2004 017 864 U1, DE 20 2004 017 862 U1, DE 20 2004 017 860 U1, DE 20 2004 017 861 U1 offenbaren Infusionssets, bei denen eine flexible Führungsnadel mittels verschiedener Rückziehvorrichtungen durch den Medikamentenkanal aus dem Infusionsset herausgezogen wird, wobei die Führungsnadel durch die Begrenzungswandungen des Medikamentenkanals um mehr als 90° umgelenkt wird und in der Rückziehvorrichtung weiter verformt wird. Anschliessend wird die Rückziehvorrichtung mit der in dieser befindlichen Führungsnadel von dem Infusionsset entfernt. Dadurch, dass die Führungsnadel in der Rückziehvorrichtung aufgenommen wird, besteht bei diesen Infusionssets zwar keine Verletzungsgefahr für den Bediener. Indes birgt die hier praktizierte Umlenkung der Führungsnadel im Medikamentenkanal jedoch die Gefahr, dass an den Begrenzungswandungen des Medikamentenkanals Abrieb entsteht, welcher mit dem Medikament in das subkutane Gewebe des Patienten eingetragen werden kann. Auch ist die Herstellung praxistauglicher Ausführungsformen gemäss diesem Konzept schwierig und teuer.

### DARSTELLUNG DER ERFINDUNG

Es stellt sich daher die Aufgabe, eine Anordnung zum Zuführen einer Flüssigkeit in den Körper eines Patienten oder zum Entnehmen einer Flüssigkeit aus dem Körper eines Patienten zur Verfügung zu stellen, welche die Nachteile des Standes der Technik nicht aufweist oder diese zumindest teilweise vermeidet.

Diese Aufgabe wird durch die Anordnung gemäss Patentanspruch 1 gelöst.

Demgemäss umfasst die erfindungsgemässe Anordnung zum bevorzugterweise subkutanen Zuführen einer Flüssigkeit, wie z.B. eines flüssigen Medikaments oder einer diagnostischen Substanz, in den Körper eines Patienten bzw. zum Abführen einer Flüssigkeit aus dem Körper eines Patienten ein Kanülengehäuse, welche eine flexible Kanüle (Softkanüle) trägt. Die Kanüle steht von einer Aussenfläche des Kanülengehäuses ab und wird von einer Führungsnadel, bevorzugterweise aus Metall oder Kunststoff, gestützt, welche die Kanüle entlang ihres Kanülenkanals durchsetzt und bevorzugterweise am Austrittsende der Kanüle, welches dem Kanülengehäuse abgewandt ist, mit einer Spitze austritt. Das Kanülengehäuse weist einen Kanal auf, über welchen, nach einem Entfernen der Führungsnadel aus dem Kanülenkanal, eine Flüssigkeit, z.B. ein flüssiges Medikament oder eine flüssige diagnostische Substanz, zur Eintrittsöffnung der Kanüle geführt werden kann und von dort durch den Kanülenkanal zu einer Austrittsöffnung am Austrittsende der Kanüle, oder aber eine Flüssigkeit, insbesondere eine Körperflüssigkeit wie Blut, von der Kanüle nach aussen abgeführt werden kann. Dabei weisen die Begrenzungswandungen des Kanals an einer Stelle, welche der Eintrittsöffnung der Kanüle direkt und in Verlängerung der Kanülenlängsachse gegenüberliegt, ein Dichtelement, bevorzugterweise ein Septum auf, durch welches hindurch die Führungsnadel aus dem Kanülengehäuse austritt. Des Weiteren umfasst die erfindungsgemässe Anordnung ein Umlenkelement zur Bewirkung einer Umlenkung der Führungsnadel. Das Umlenkelement ist gegenüberliegend dem Austrittsort der Führungsnadel aus dem zuvor erwähnten Dichtelement des Kanülengehäuses angeordnet und bildet eine Führung, in welcher die Führungsnadel axial verschieblich geführt und dabei umgelenkt wird. Dabei weist die Führungsnadel zwischen dem Austrittsende der Kanüle und ihrem Eintritt in das Umlenkelement einen geradlinigen Verlauf auf, welcher im Eintrittsbereich des Umlenkelements durch die Führung übernommen wird, bis dann die eigentliche Umlenkung der Führungsnadel erfolgt. Hier- durch lässt sich sicherstellen, dass in den zwischen Kanüle und Umlenkelement angeordneten Abschnitt der Führungsnadel trotz der Umlenkung im Umlenkelement keinerlei Biegemomente eingeleitet werden können, welche zu Dichtungs- oder Abriebproblemen führen könnten. Zudem umfasst die erfindungsgemässe Anordnung eine Rückziehvorrichtung für die Führungsnadel, mittels welcher die Führungsnadel durch das Umlenkelement hindurch unter Umlenkung in demselben soweit zurückgezogen werden kann, bis sie aus dem Kanülenkanal austritt und die Eintrittsöffnung der Kanüle freigibt.

Durch die Erfindung wird die Bereitstellung von Anordnungen zur subkutanen Zuführung von flüssigen Medikamenten in den Körper eines Patienten möglich, welche ein Höchstmass an medizinischer Sicherheit bieten, kostengünstig in der Herstellung sind und zudem einfach und sicher durch den Patienten selbst zu applizieren sind.

In einer bevorzugten Ausführungsform der Anordnung bilden das Kanülengehäuse, das Umlenkelement und die Rückziehvorrichtung eine untrennbare Einheit, innerhalb welcher die Führungsnadel nach dem Zurückziehen derselben vor dem Zugriff eines Bedieners geschützt verbleibt. Hierdurch lassen sich besonders kostengünstige erfindungsgemässe Anordnungen bereitstellen, da auf mehrere, einzelne Funktionseinheiten bildende Gehäuse verzichtet werden kann.

In einer anderen bevorzugten Ausführungsform der Anordnung ist die Rückziehvorrichtung derartig ausgestaltet, dass mit ihr die Führungsnadel in eine Rückzugendposition zurückgezogen werden kann, in welcher die Führungsnadel vollständig aus dem Dichtelement herausgezogen ist und die Spitze der Führungsnadel innerhalb der Führung des Umlenkelements bzw. innerhalb der Rückziehvorrichtung angeordnet ist. Gleichzeitig sind das Kanülengehäuse, das Umlenkelement und die Rückziehvorrichtung derartig miteinander verbunden, dass diese für den Fall, dass die Führungsnadel in einer anderen als der bestimmungsgemässen Rückzugendposition angeordnet ist, eine untrennbare Einheit bilden. Befindet sich die Führungsnadel indes, nach dem Zurückziehen derselben mit der Rückziehvorrichtung, in der Rückzugendposition, so kann zumindest die Rückziehvorrichtung und gegebenenfalls zusätzlich auch das Umlenkelement vom Kanülengehäuse entfernt werden. Je nach Ausgestaltung ist die Spitze der Führungsnadel nach dem Entfernen der Rückziehvorrichtung und allenfalls auch des Umlenkelements vom Kanülengehäuse vor einem Zugriff eines Benutzers geschützt innerhalb der Rückziehvorrichtung angeordnet oder für den Fall, dass die Rückziehvorrichtung und dass Umlenkelement eine zusammenhängende, untrennbare Einheit bilden, was bevorzugt ist, bevorzugterweise innerhalb der Führung des Umlenkelements angeordnet. Hierdurch ergibt sich der Vorteil, dass lediglich das für die Zuführung von flüssigen Medikamenten erforderliche Kanülengehäuse am Körper des Patienten verbleiben muss, wodurch der Tragekomfort deutlich gesteigert werden kann, da das Kanülengehäuses als solches relativ klein und zudem flach bauen kann.

In einer weiteren bevorzugten Ausführungsform der Anordnung umfasst die Rückziehvorrichtung zwei entlang von Führungen relativ zueinander bewegbare Bauteile. Dabei ist das erste Bauteil gegenüber dem Kanülengehäuse feststehend, während das zweites Bauteil gegenüber dem Kanülengehäuse bewegbar und derartig mit der Führungsnadel gekoppelt ist, dass bei einem Bewegen der beiden Bauelemente relativ zueinander die Führungsnadel durch die Bewegung des zweiten Bauteils zurückgezogen wird.

Sind dabei die beiden Bauteile als bevorzugterweise translatorisch zusammenschiebbare Bauelemente ausgebildet, was bevorzugt ist, so sind konstruktiv einfache und kostengünstige Lösungen möglich.

Dabei ist es weiter bevorzugt, wenn die Zusammenschieberichtung der beiden Bauteile quer zur Richtung der Längserstreckung der Kanüle liegt, bevorzugterweise senkrecht zu dieser orientiert ist und/oder derartig orientiert ist, dass sie bei bestimmungsgemäss am Körper einer Person applizierter Anordnung im wesentlichen parallel zur Hautoberfläche im Bereich der Applikationsstelle verläuft, da hierdurch eine Krafteinwirkung auf das Kanülengehäuse in axialer Richtung der Kanüle, welche zu einem Zurück- bzw. Wiederherausziehen der applizierten Kanüle aus dem Gewebe führen könnte, beim Zurückziehen der Führungsnadel verhindert wird.

Ebenfalls bevorzugt ist es bei den Ausführungsformen, bei denen die beiden Bauteile als zusammenschiebbare Bauelemente ausgebildet sind, dass eines der beiden Bauteile zwei in einer Richtung quer zur Zusammenschieberichtung der Bauteile voneinander beabstandete Griffflächen zur Auflage der jeweiligen Fingerkuppen von Zeigefinger und Mittelfinger eines Benutzers aufweist. Gleichzeitig weist das andere der beiden Bauteile eine diesen beiden Griffflächen in Zusammenschieberichtung etwa mittig gegenüberliegende Grifffläche zur Auflage der Kuppe des Daumens des Benutzers auf, so dass das Zusammenschieben der beiden Bauteile durch eine der Betätigung einer Spritze ähnliche Fingerbewegung erfolgen kann. Hierdurch lassen sich relativ grosse Betätigungskräfte zum Zurückziehen der Führungsnadel gut kontrollierbar bereitstellen.

Sind dabei die beiden Bauteile als gegeneinander verdrehbare Bauelemente ausgebildet, was alternativ bevorzugt ist, so lassen sich relativ grosse Zurückziehkräfte und Zurückziehwege realisieren.

Dabei ist es bevorzugt, wenn die Verdrehachse der beiden Bauteile parallel zur Richtung der Längserstreckung der Kanüle verläuft und/oder derartig orientiert ist, dass sie bei bestimmungsgemäss am Körper einer Person applizierter Anordnung im wesentlichen senkrecht zur Hautoberfläche im Bereich der Applikationsstelle verläuft, da sich hierdurch eine Krafteinwirkung auf das Kanülengehäuse in axialer Richtung der Kanüle beim Zurückziehen der Führungsnadel, welche zu den bereits zuvor erwähnten Problemen führen könnte, vermeiden lässt.

Mit Vorteil sind bei den Ausführungsformen der erfindungsgemässen Anordnung, bei denen die Rückziehvorrichtung zwei entlang von Führungen relativ zueinander bewegbare Bauteile umfasst, welche Zwecks Bewirkung der Rückzugsbewegung der Führungsnadel zusammen geschoben oder gegeneinander verdreht werden, diese beiden Bauteile derartig ausgebildet, dass sie sich beim Zusammenschieben oder Gegeneinanderverdrehen, bevorzugterweise in einer Bewegungsendposition, in welcher die Führungsnadel maximal zurückgezogen ist, miteinander verrasten, derart, dass die Rückzugsbewegung irreversibel ist. Hierdurch lassen sich Fehlmanipulationen nach erfolgter Rückzugsbewegung der Führungsnadel sicher verhindern, was insbesondere bei erfindungsgemässen Anordnungen mit abnehmbarer Rückziehvorrichtung von Bedeutung ist, da eine umgekehrte Bewegungsrichtung der Führungsnadel bei vom Kanülengehäuse entfernter Rückziehvorrichtung die Spitze der Führungsnadel zugänglich machen könnte und eine entsprechende Verletzungsgefahr begründen könnte.

In noch einer weiteren bevorzugten Ausführungsform der erfindungsgemässen Anordnung ist die Rückziehvorrichtung derartig ausgestaltet, dass mit ihr die Führungsnadel in eine Rückzugendposition zurückgezogen werden kann, in welcher die Führungsnadel vollständig aus dem Dichtelement herausgezogen ist und die Spitze der Führungsnadel innerhalb der Führung des Umlenkelements bzw. innerhalb der Rückziehvorrichtung angeordnet ist. Das Kanülengehäuse, das Umlenkelement und die Rückziehvorrichtung sind derart miteinander verbunden, dass diese für den Fall, dass die Führungsnadel in einer anderen als der bestimmungsgemässen Rückzugendposition angeordnet ist, eine untrennbare Einheit bilden, und für den Fall, dass sich die Führungsnadel nach dem Zurückziehen in der Rückzugendposition befindet, zumindest die Rückziehvorrichtung und gegebenenfalls zusätzlich auch das Umlenkelement vom Kanülengehäuse entfernt werden kann. Dabei ist die Spitze der Führungsnadel nach dem Entfernen der Rückziehvorrichtung und allenfalls auch des Umlenkelements vom Kanülengehäuse vor einem Zugriff eines Benutzers geschützt innerhalb der Rückziehvorrichtung bzw. innerhalb der Führung des Umlenkelements angeordnet. Die Rückziehvorrichtung umfasst zwei entlang von Führungen relativ zueinander bewegbare Bauteile, von denen das erste gegenüber dem Kanülengehäuse feststehend ist, während das zweite Bauteil gegenüber dem Kanülengehäuse bewegbar ist und derartig mit der Führungsnadel gekoppelt ist, dass bei einem Bewegen der beiden Bauteile relativ zueinander die Führungsnadel durch die Bewegung des zweiten Bauteils zurückgezogen wird. Dabei ist das erste Bauteil über einen Befestigungsmechanismus entfernbar am Kanülengehäuse befestigt, welcher durch das zweite Bauteil verriegelt ist und erst nach einem Bewegen der beiden Bauteile relativ zueinander in eine Bewegungsendposition, in welcher die Führungsnadel in der Rückzugendposition angeordnet ist, freigegeben wird.

Dabei ist es bevorzugt, wenn das Kanülengehäuse einen Anschlussport für eine Leitung zur Zuführung einer Flüssigkeit in den Kanal oder Abführung einer Flüssigkeit aus dem Kanal aufweist, welcher erst nach einem Entfernen des ersten Bauteils zugänglich ist.

Solche Anordnungen bieten ein Maximum an konstruktiver Sicherheit gegen Fehlmanipulationen beim Applizieren und weisen zudem auch hygienetechnische Vorteile auf, da der Anschlussport bis kurz vor der Ankopplung der Leitung durch das erste Bauteil geschützt wird.

In noch einer weiteren bevorzugten Ausführungsform der Anordnung ist diejenige Aussenseite des Kanülengehäuses, auf welcher die Kanüle von diesem absteht, im Wesentlichen eben ausgebildet. Auch ist es bevorzugt, wenn die Kanüle im Wesentlichen senkrecht von dieser Aussenfläche absteht. Solche Ausführungsformen eignen sich besonders gut als Infusionsset zur Zuführung von flüssigen Medikamenten in das subkutane Gewebe der Bauchdecke eines Patienten. Es sind jedoch auch Ausführungsformen vorgesehen, bei denen die Kanüle nicht senkrecht von der Aussenflächg absteht, z.B. unter einem Winkel von 30°.

In noch einer weiteren bevorzugten Ausführungsform der Anordnung trägt die Aussenseite des Kanülengehäuses, auf welcher die Kanüle von diesem absteht, eine Klebeschicht, welche bevorzugterweise von einem Pflaster gebildet wird, mit welcher das Kanülengehäuse beim Einstechen der Kanüle auf die Haut des Patienten aufgeklebt werden kann. Hierdurch ergibt sich eine gute und positionsgenaue Fixierung des Kanülengehäuses am Körper.

In noch einer weiteren bevorzugten Ausführungsform der Anordnung wird die Führungsnadel in der Führung des Umlenkelements um etwa 90° umgelenkt, so dass sich das Ende der Führungsnadel, welches mit der Rückziehvorrichtung gekoppelt ist, im Wesentlichen senkrecht zur Kanülenlängsachse erstreckt. Insbesondere bei den zuvor beschriebenen Ausführungsformen mit Rückziehvorrichtungen mit zwei zusammenschiebbaren Bauteilen zur Bewirkung der Rückzugsbewegung ergibt sich hierdurch der Vorteil, dass die Zusammenschieberichtung der beiden Bauteile praktisch zwangsläufig senkrecht zur Richtung der Längserstreckung der Kanüle orientiert ist, so dass beim Betätigen der Rückziehvorrichtung eine Krafteinwirkung auf das Kanülengehäuse in axialer Richtung der Kanüle, welche zu einem Zurück- bzw. Wiederherausziehen der applizierten Kanüle aus dem Gewebe führen könnte, verhindert wird. Es sind jedoch auch Ausführungsformen vorgesehen, bei denen die Führungsnadel um mehr oder weniger als 90° umgelenkt wird, z.B. um 30°.

Bei Verwendung der Anordnung nach einem der vorangehenden Ansprüche zum Zuführen eines flüssigen Medikaments oder einer flüssigen diagnostischen Substanz, bevorzugterweise von Insulin, in den Körper eines Patienten treten die Vorteile der Erfindung besonders deutlich zu Tage.

### KURZE BESCHREIBUNG DER ZEICHNUNGEN

Weitere bevorzugte Ausführungen der Erfindung ergeben sich aus den abhängigen Ansprüchen und aus der nun folgenden Beschreibung anhand der Figuren. Dabei zeigen:
Fig. 1 eine Seitenansicht einer ersten erfindungsgemässen Anordnung vor der Applikation;
Fig. 2 eine Seitenansicht der Anordnung aus Fig. 1 nach der Applikation;
Fig. 3 einen Längsschnitt durch die in Fig. 1 dargestellte Anordnung;
Fig. 4 einen Längsschnitt durch die in Fig. 2 dargestellte Anordnung;
Fig. 5 eine perspektivische Draufsicht auf die in Fig. 1 dargestellte Anordnung;
Fig. 6 eine perspektivische Draufsicht auf die in Fig. 2 dargestellte Anordnung;
Fig. 7 eine Seitenansicht der Anordnung aus Fig. 1 nach der Applikation mit vom Kanülengehäuse entfernter Rückziehvorrichtung;
Fig. 8 eine Seitenansicht des applizierten Kanülengehäuses aus Fig. 7 verbunden mit einem Zuführungsschlauch;
die Figuren 9a bis 9c Längsschnitte durch die in Fig. 1 gezeigte Anordnung während der Herstellung in verschiedenen Herstellungsstadien;
Fig. 10 eine perspektivische Draufsicht auf den feststehenden Teil der Rückziehvorrichtung der Anordnung aus Fig. 1;
Fig. 11 eine perspektivische Draufsicht auf den beweglichen Teil der Rückziehvorrichtung der Anordnung aus Fig. 1;
Fig. 12 eine perspektivische Draufsicht auf eine zweite erfindungsgemässe Anordnung vor der Applikation;
Fig. 13 eine perspektivische Draufsicht auf die Anordnung aus Fig. 12 nach der Applikation;
Fig. 14 eine perspektivische Draufsicht auf eine dritte erfindungsgemässe Anordnung nach der Applikation;
Fig. 15 eine perspektivische Draufsicht auf die Anordnung aus Fig. 14 vor der Applikation;
Fig. 16 einen Längsschnitt durch die in Fig. 15 dargestellte Anordnung;
Fig. 17 einen Längsschnitt durch die in Fig. 14 dargestellte Anordnung;
Fig. 18 eine perspektivische Draufsicht auf eine vierte erfindungsgemässe Anordnung nach der Applikation;
Fig. 19 einen Längsschnitt durch die in Fig. 18 dargestellte Anordnung, jedoch vor der Applikation;
Fig. 20 einen Längsschnitt durch die in Fig. 18 dargestellte Anordnung; und
die Figuren 21a bis 21d schematische Seitenansichten der in Fig. 19 gezeigten Anordnung während der Herstellung in verschiedenen Herstellungsstadien.

### WEGE ZUR AUSFÜHRUNG DER ERFINDUNG

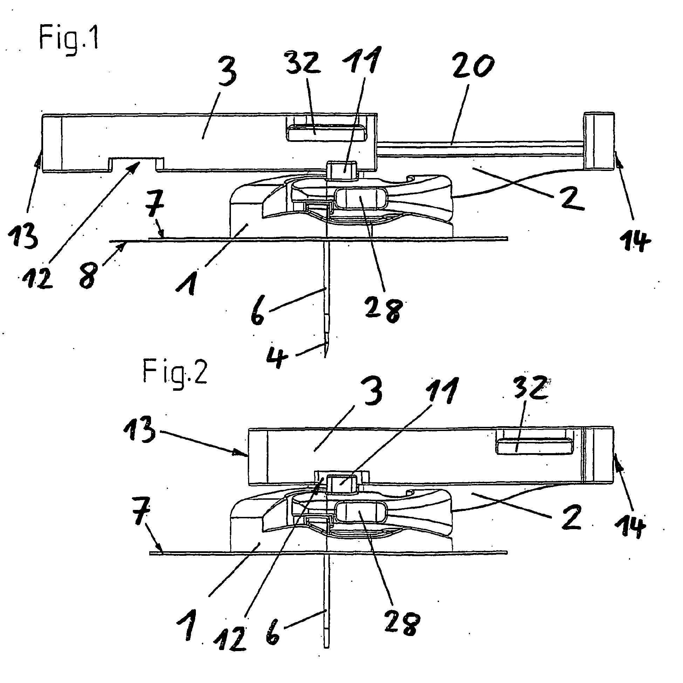

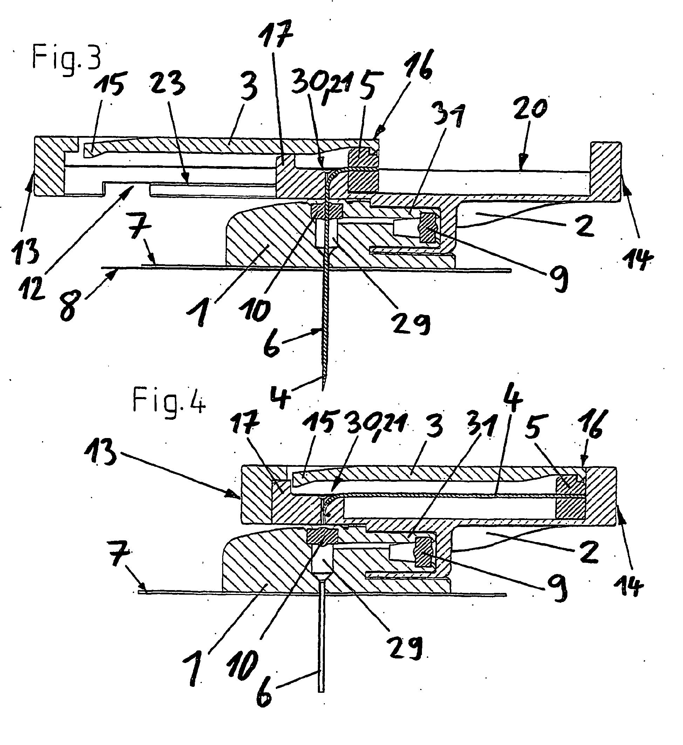

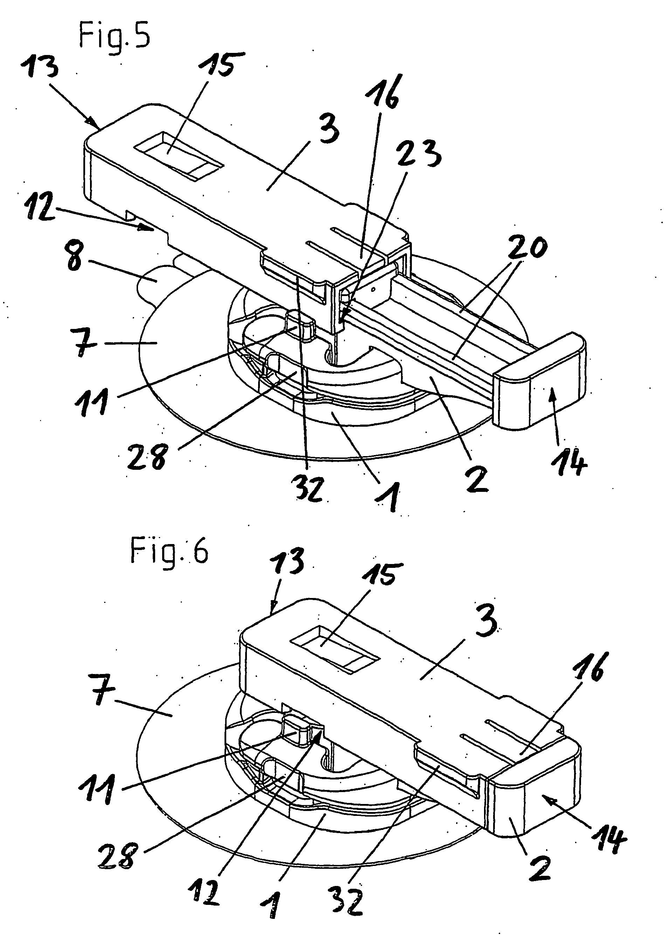

Eine erste bevorzugte Ausführungsform der erfindungsgemässen Anordnung in Form eines Infusionssets ist in den Figuren 1 und 2 in der Seitenansicht, in den Figuren 3 und 4 im Längsschnitt und in den Figuren 5 und 6 in einer perspektivischen Draufsicht dargestellt, und zwar einmal in einem unbenutzten Ursprungszustand (Figuren 1, 3 und 5) und einmal in einem Zustand nach der Applikation auf dem Körper eines Patienten (Figuren 2, 4 und 6). Wie in Zusammenschau dieser Figuren zu erkennen ist, umfasst das Infusionsset ein Kanülengehäuse 1, welches auf seiner Unterseite eine vom Kanülengehäuse 1 senkrecht abstehende Kanüle 6 aus einem flexiblen Material trägt. Die Unterseite des Kanülengehäuses 1 wird von einem Pflaster 7 gebildet, dessen Klebefläche im Ursprungszustand mit einem Pflasterschutzpapier 8 bedeckt ist. Die Kanüle 6 ist im Ursprungszustand in ihrem Kanülenkanal von einer Führungsnadel 4 durchsetzt, welche am freien Ende (Austrittsende) der Kanüle 6 zur Erleichterung des Einstechens in das Gewebe eines Patienten eine Spitze bildet und die flexible Kanüle 6 beim Einstechen in das Gewebe stützt. Das Kanülengehäuse 1 bildet in seinem Inneren einen Kanal 29, welcher sich zwischen der Kanüle 6 und einem einen Teil einer Seitenwand des Kanülengehäuses 1 bildenden ersten Septum 9 erstreckt, so dass ein flüssiges Medikament vom Septum 9 zur Eintrittsöffnung der Kanüle 6 geführt werden kann. Der Eintrittsöffnung der Kanüle 6 direkt und in geradliniger Verlängerung des Kanülenkanals gegenüberliegend ist der Kanal 29 von einem zweiten Septum 10 begrenzt, welches einen Teil der Oberseite des Kanülengehäuses 1 bildet und durch welches hindurch im Ursprungszustand die Führungsnadel 4 an der Oberseite aus dem Kanülengehäuse 1 austritt. Angeordnet auf dem Kanülengehäuse 1 ist eine Rückziehvorrichtung 2, 3, 5 zum Herausziehen der Führungsnadel 4 aus der Kanüle 6 und dem zweiten Septum 10 nach erfolgter Applikation des Infusionssets. Die Rückziehvorrichtung 2, 3, 5 besteht aus einem feststehendes Gehäuseteil 2, einem beweglichen Gehäuseteil 3 und einem mit der Führungsnadel 4 verbundenen Nadelmitnehmer 5. Der feststehende Gehäuseteil 2 ist mit dem Kanülengehäuse 1 über Schnapparme 28 lösbar verbunden und der bewegliche Gehäuseteil 3 ist am festehenden Gehäuseteil 2 mittels Führungsschienen 20, 23 gleitend zusammenschiebbar gelagert. Dabei bildet das dem beweglichen Gehäuseteil 3 zugewandte Ende des festehenden Gehäuseteils 2 gegenüberliegend dem Ort, an welchem im Ursprungszustand die Führungsnadel 4 aus dem zweiten Septum 10 des Kanülengehäuses 1 austritt, ein anspruchsgemässes Umlenkelement 30 mit einer Führung 21, in welcher die Führungsnadel 4 in Richtung zum Nadelmitnehmer 5 hin gesehen axial verschieblich zuerst in Verlängerung des Verlaufs des Kanülenkanals geführt wird und sodann um 90° umgelenkt wird.

Zum Applizieren des Infusionssets am Körper eines Patienten wird bei dem im Ursprungszustand befindlichen Infusionsset (Figuren 1, 3 und 5) das Pflasterschutzpapier 8 von der Klebefläche des Pflasters 7 abgezogen und anschliessend das Infusionsset mit der das Pflaster 7 tragenden Unterseite des Kanülengehäuses 1 fest auf die Haut des Patienten gedrückt, wobei die Kanüle 4 durch die Haut in das subkutane Gewebe des Patienten eingestochen wird und das Kanülengehäuse 1 durch das Pflaster 7 auf der Haut befestigt wird. Beim Einstechen wird die flexible Kanüle 6 durch die Führungsnadel 4 gestützt, welche auch die eigentliche Einstechspitze bildet. Das Applizieren kann grundsätzlich von Hand oder mit einer Applikationshilfe erfolgen, an welcher das Infusionsset mittels der dafür am bewegbaren Gehäuseteil 3 gebildeten Befestigungsschultern 32 befestigt werden kann.

Nach dem Applizieren des Infusionssets auf der Haut werden das feststehende Gehäuseteil 2 und das bewegliche Gehäuseteil 3 der Rückziehvorrichtung 2, 3, 5 durch Druck mit zwei Fingern auf deren Stirnflächen 13, 14 zusammengeschoben, wobei die Führungsnadel 4 unter formschlüssiger Mitnahme des Nadelmitnehmers 5 durch das bewegliche Gehäuseteil 3 durch das Umlenkelement 30 des festehenden Gehäuseteils 2 soweit zurückgezogen wird, bis sie vollständig aus dem Kanülengehäuse 1 entfernt ist und die kontaminierte Spitze der Führungsnadel 4 geschützt in der Führung 21 des Umlenkelements 30 angeordnet ist. Diese Situation ist in Fig. 4 dargestellt.

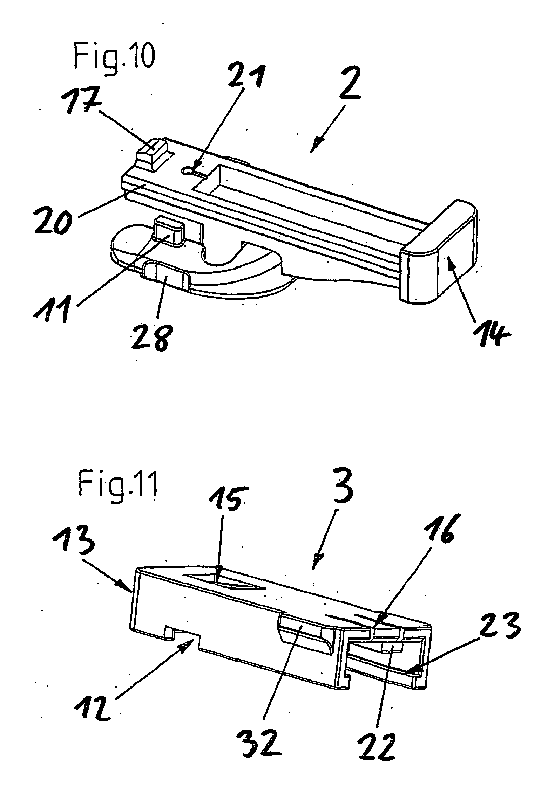

Wie in Zusammenschau mit den Figuren 10 und 11 zu erkennen ist, welche jeweils eine perspektivische Darstellung des feststehenden Gehäuseteils 2 (Fig. 10) und des beweglichen Gehäuseteils 3 (Fig. 11) zeigen, erfolgt die formschlüssige Mitnahme des Nadelmitnehmers 5 über eine Mitnahmeschulter 22 am beweglichen Gehäuseteil 3. Nachdem die beiden Gehäuseteile 2, 3 vollständig zusammengeschoben sind, sich die Führungsnadel 4 also in ihrer Rückzugsendposition befindet, verrastet der vom beweglichen Gehäuseteil 3 gebildete Schnapper 15 mit einer vom feststehenden Gehäuseteil 2 gebildeten Rastnase 17, so dass der Zusammenschiebevorgang irreversibel ist. Es sind auch Ausführungsformen vorgesehen, bei denen zusätzlich bereits auf Zwischenpositionen eine irreversible Verrastung erfolgt, so dass bereits bei teilweise zusammengeschobenen Gehäuseteilen 2, 3 kein Zurückschieben der Führungsnadel 4 in die Kanüle 6 mehr möglich ist.

Nach dem vollständigen Zurückziehen der Führungsnadel 4 kann die Rückziehvorrichtung 2, 3, 5 vom Kanülengehäuse 1 getrennt werden, indem die Schnapparme 28 unter Eintreten von an diesen gebildeten Sicherheitsnocken 11 in Aussparungen 12 am beweglichen Gehäuseteil 3 nach innen bewegt werden, wodurch die Verbindung zwischen dem Kanülengehäuse 1 und dem feststehenden Gehäuseteil 2 aufgehoben wird. In allen anderen Relativpositionen der beiden Gehäuseteile 2, 3 zueinander verhindern die Sicherheitsnocken 11 durch Anstehen an den Seitenwänden des beweglichen Gehäuseteils 3 ein Nachinnenbewegen der Schnapparme 28, so dass ein Trennen von Kanülengehäuse 1 und Rückziehvorrichtung 2, 3, 5 ausschliesslich bei vollständig zurückgezogener Führungsnadel 4 möglich ist.

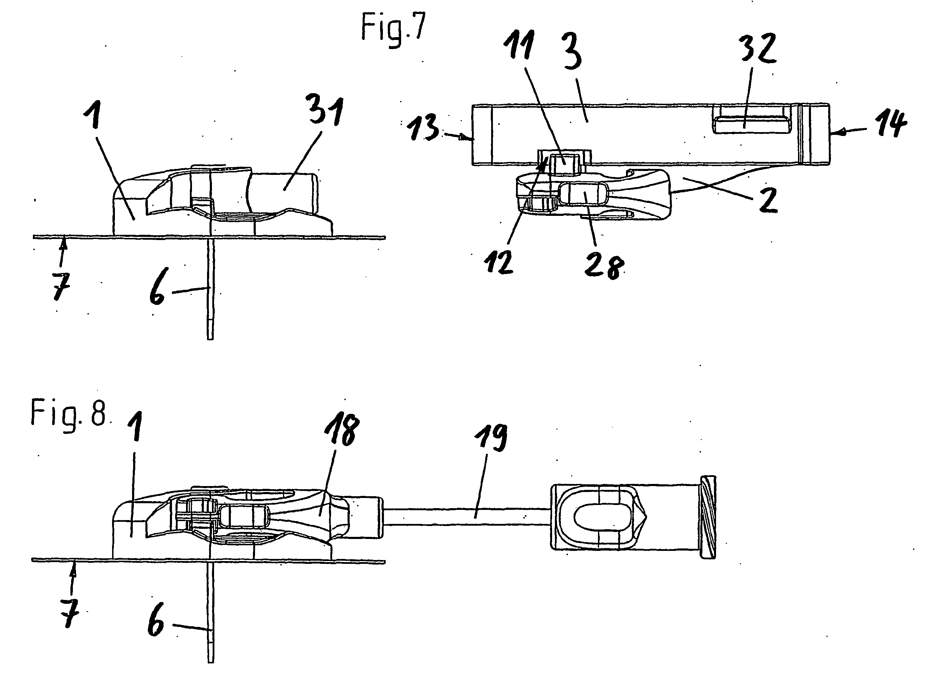

Die Situation nach dem Trennen von Rückziehvorrichtung 2, 3, 5 und Kanülengehäuse 1 ist in der Seitenansicht in Fig. 7 gezeigt. Wie zu erkennen ist, wird durch das Entfernen der Rückziehvorrichtung 2, 3, 5 der Anschlussport 31 des Kanülengehäuses 1 zugänglich, so dass ein Zuführungsschlauch 19 mit einem geeigneten Konnektor 18 an das Kantilengehäuse 1 angeschlossen werden kann, zur Zuführung flüssiger Medikamente über den Kanal 29 zur Kanüle 6. Diese Situation ist in Fig. 8 dargestellt.

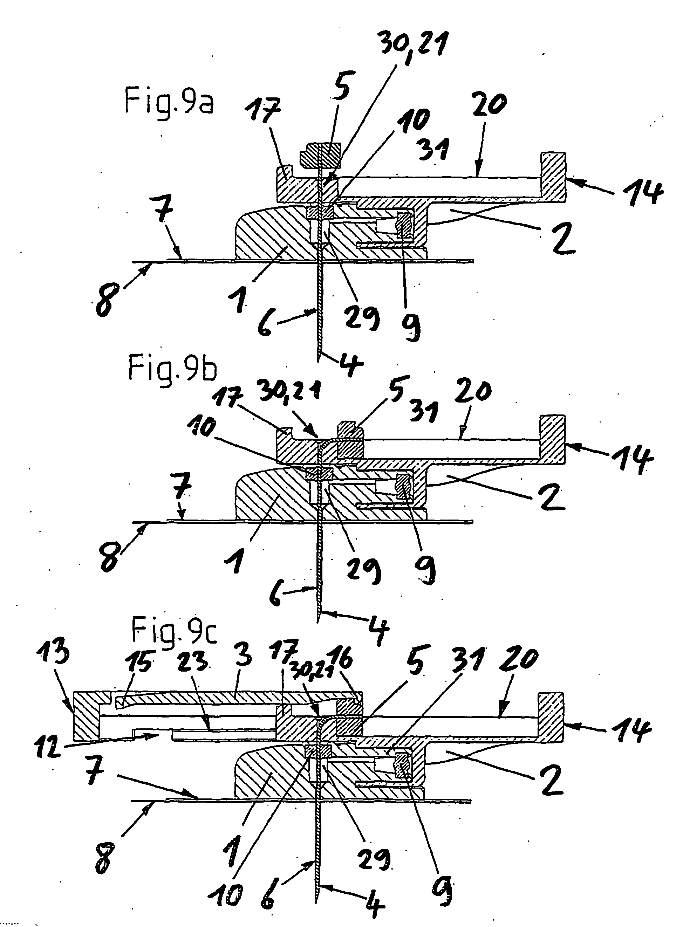

Die Figuren 9a bis 9c zeigen Längsschnitte durch die in Fig. 1 gezeigte Anordnung während derer Herstellung in verschiedenen Herstellungsstadien. Wie in Fig. 9a erkennbar ist, wird nach dem Befestigen des feststehenden Gehäuseteils 2 der Rückziehvorrichtung 2, 3, 5 auf dem Kanülengehäuse 1 die an ihrem Ende mit dem Nadelmitnehmer 5 verbundene, gerade Führungsnadel 4 durch die Führung 21 des vom feststehenden Gehäuseteil 2 gebildeten Umlenkelements 30 und durch das zweite Septum 10 hindurch in den Kanülenkanal der flexiblen Kanüle 6 eingeführt, bis die Spitze der Führungsnadel 4 aus der Austrittöffnung der Kanüle 6 heraussteht. Sodann wird die Führungsnadel 4 mit Hilfe des Nadelmitnehmers 5 an der Umlenkstelle des Umlenkelements 30 um 90° umgebogen, so dass der Nadelmitnehmer 5 bestimmungsgemäss in einer für diesen vorgesehenen Ausnehmung im feststehenden Gehäuseteil 2 der Rückziehvorrichtung 2, 3, 5 zu liegen kommt. Diese Situation ist in Fig. 9b dargestellt. Anschliessend wird das bewegliche Gehäuseteil 3 mit seinen Führungsschienen 23 auf die Führungsschienen 20 des feststehenden Gehäuseteils 2 aufgeschoben, bis die Rastnase 16 in einem hierfür vorgesehenen Absatz am Nadelmitnehmer 5 einrastet und das bewegliche Gehäuseteil 3 nicht mehr entfernt werden kann. Diese Situation ist in Fig. 9c dargestellt und ist identisch zu der in den Figuren 1, 3 und 5 dargestellten Ursprungssituation.

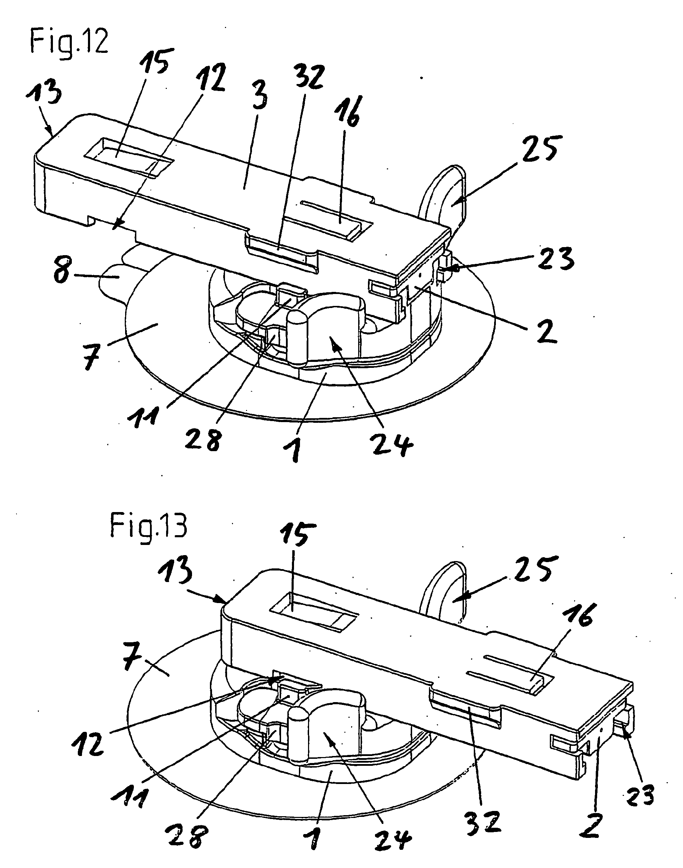

Die Figuren 12 und 13 zeigen eine alternative Ausführungsform des zuvor beschriebenen Infusionssets, einmal in einem unbenutzten Ursprungszustand (Fig. 12) und einmal in einem Zustand nach der Applikation auf dem Körper eines Patienten (Fig. 13), bei der die Bedienung der Rückziehvorrichtung 2, 3, 5 mit drei Fingern ähnlich der Betätigung einer Spritze erfolgen kann. Hierzu weist der feststehende Gehäuseteil 2 zwei Griffflächen 24, 25 für die Kuppen von Zeigefinger und Mittelfinger auf und der bewegliche Gehäuseteil 3 eine Stirnfläche 13, auf welche mit dem Daumen Druck ausgeübt werden kann.

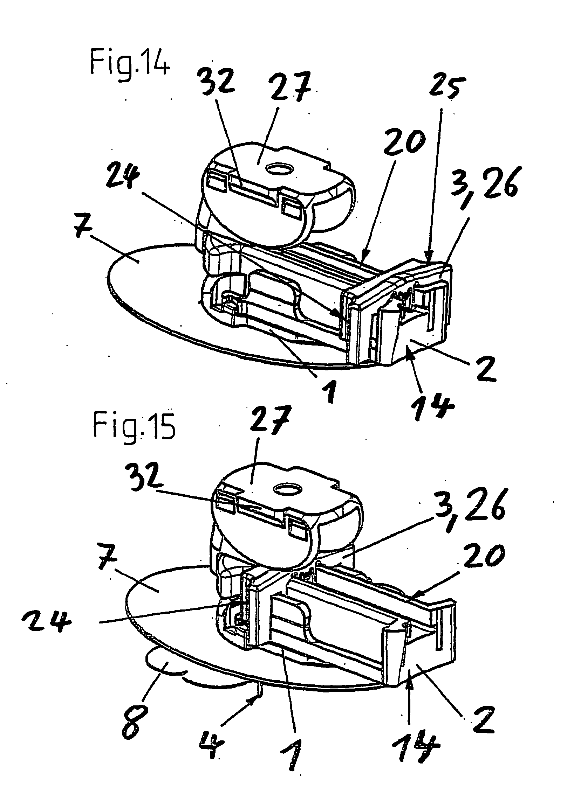

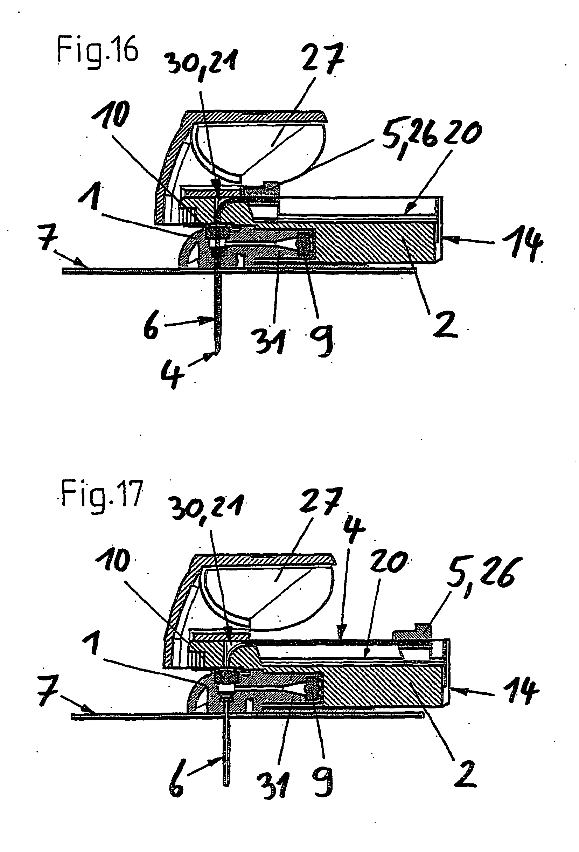

Die Figuren 14 bis 17 zeigen eine dritte Ausführungsform der erfindungsgemässen Anordnung in Form eines Infusionssets in perspektivischen Draufsichten und in Längsschnitten, und zwar einmal in einem unbenutzten Ursprungszustand (Figuren 15 und 16) und einmal in einem Zustand nach der Applikation auf dem Körper eines Patienten (Figuren 14 und 17). Wie in Zusammenschau dieser Figuren zu erkennen ist, weist das Infusionsset einen ähnlichen Aufbau wie das zuvor beschriebene auf, jedoch mit dem wesentlichen Unterschied, dass hier zum Zurückziehen der Führüngsnadel 4 nicht das bewegbare Gehäuseteil 3 und das feststehende Gehäuseteil 2 zusammengeschoben werden, sondern das bewegbare Gehäuseteil 3 als in Führungen 20 entlang dem feststehenden Gehäuseteil 2 verschiebbarer Schieber 26 ausgebildet ist, welcher gleichzeitig auch den Nadelmitnehmer 5 bildet und bei der Montage in die Führungen 20 eingeschnappt wird. Ein weiterer Unterschied besteht darin, dass der feststehende Gehäuseteil 2 einen auf diesen aufgesteckten knaufartigen Griffteil 27 aufweist, welcher zur Applikation ergriffen werden kann oder mittels der daran ausgebildeten Befestigungsschultern 32 an einer Applikationsvorrichtung befestigt werden kann. Hier nicht explizit beschriebene aber mit Bezugsziffern versehene Elemente haben die gleiche Funktion wie die Bauteile mit entsprechender Bezugsziffer bei den vorher beschriebenen Ausführungen.

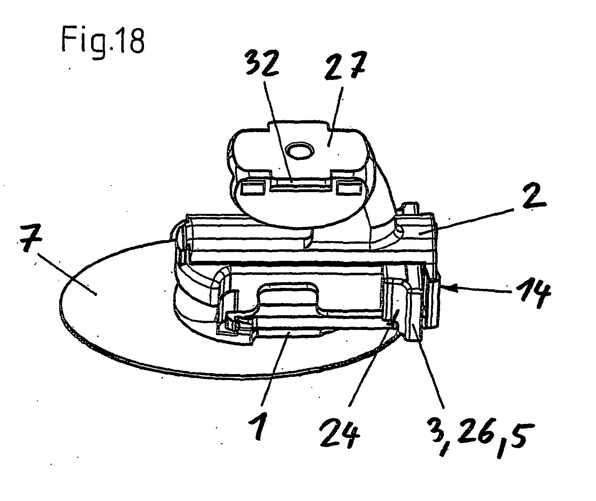

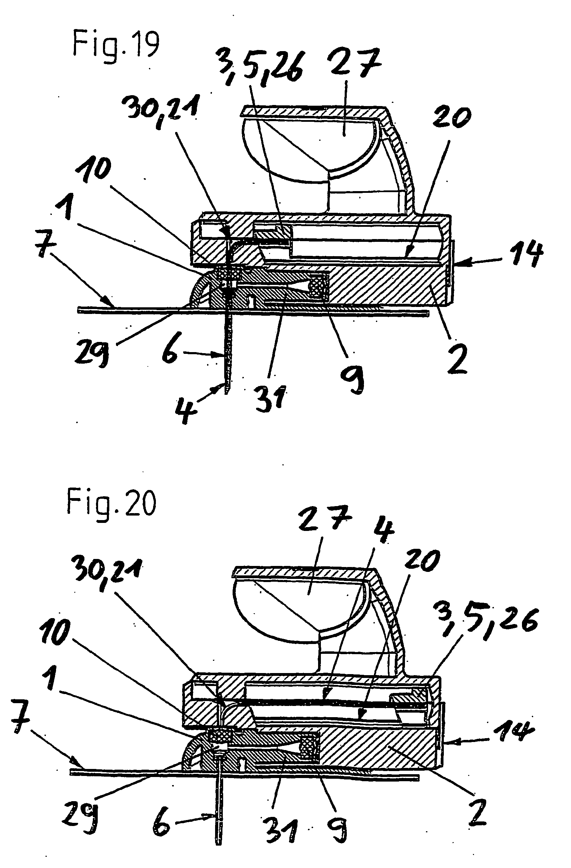

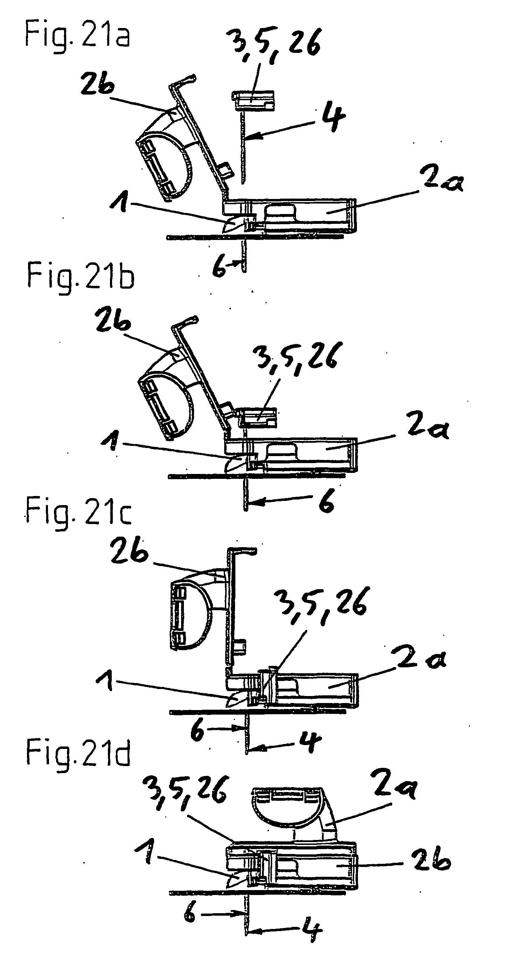

Die Figuren 18 bis 20 zeigen eine vierte Ausführungsform der erfindungsgemässen Anordnung in Form eines Infusionssets in einer perspektivischen Draufsicht und in zwei Längsschnitten, und zwar einmal in einem unbenutzten Ursprungszustand (Fig. 19) und zweimal in einem Zustand nach der Applikation auf dem Körper eines Patienten (Figuren 18 und 20). Wie in Zusammenschau dieser Figuren zu erkennen ist, weist das Infusionsset einen ähnlichen Aufbau wie das in den Figuren 14 bis 17 gezeigte auf. Ein wesentlicher Unterschied besteht jedoch darin, dass das als Schieber 26 ausgebildete bewegliche Gehäuseteil 3 hier lediglich mit zwei jeweils eine der Griffflächen 24, 25 für Zeigefinger und Mittelfinger bereitstellenden Flügeln seitlich aus dem feststehenden Gehäuseteil 2 herausschaut. Ein weiterer Unterschied besteht darin, dass die Tragstruktur des knaufartigen Griffteils 27 am anderen Ende des feststehenden Gehäuseteils 2 angeordnet ist und, wie in Zusammenschau mit den Figuren 21a bis 21d erkennbar wird, nicht als separates Teil auf das feststehende Bauteil 2 aufgesteckt ist, sondern einstückig mit diesem ausgebildet ist. Auch für diese Ausführungsform gilt, dass nicht explizit beschriebene aber mit Bezugsziffern versehene Elemente die gleiche Funktion wie die Bauteile mit entsprechender Bezugsziffer bei den vorher beschriebenen Ausführungen haben.

Die Figuren 21a bis 21d zeigen schematische Seitenansichten der in den Figuren 18 bis 20 gezeigten Anordnung während derer Herstellung in verschiedenen Herstellungsstadien. Wie zu erkennen ist, kommt hier bei der Herstellung des Infusionssets ein von zwei einstückig über eine Scharnierstelle miteinander verbundenen Teilelementen 2a, 2b gebildetes feststehendes Gehäuseteil 2 zum Einsatz, welches mit dem ersten Teilelement 2a auf dem Kanülengehäuse 1 befestigt wird, während das zweite Teilelement zur Seite geklappt ist (Fig. 21a). Sodann wird die an ihrem Ende mit dem als Schieber 26 ausgebildeten und gleichzeitig den Nadelmitnehmer 5 bildenden bewegbaren Gehäuseteil 3 verbundene, gerade Führungsnadel 4 durch die Führung 21 des vom feststehenden Gehäuseteil 2 gebildeten Umlenkelements 30 und durch das zweite Septum 10 hindurch in den Kanülenkanal der flexiblen Kanüle 6 eingeführt, bis die Spitze der Führungsnadel 4 aus der Austrittöffnung der Kanüle 6 heraussteht. Anschliessend wird die Führungsnadel 4 mit Hilfe des bewegbaren Gehäuseteils 3 an der Umlenkstelle des Umlenkelements 30 um 90° umgebogen, so dass das bewegbare Gehäuseteil 3 bestimmungsgemäss in einer für dieses vorgesehenen Ausnehmung im ersten Teilelement 2a des feststehenden Gehäuseteils 2 der Rückziehvorrichtung 2, 3, 5 zu liegen kommt (Figuren 21b und 21c). Anschliessend wird das zweite Teilelement 2b des feststehenden Gehäuseteils 2 um die Scharnierstelle herum heruntergeklappt und am ersten Teilelement 2a unlösbar eingerastet, wodurch das applikationsbereite Infusionsset wie in Fig. 21d dargestellt entsteht.

## Patentansprüche

1. Anordnung zum insbesondere subkutanen Zuführen einer Flüssigkeit in den Körper eines Patienten oder zum Abführen einer Flüssigkeit aus dem Körper eines Patienten, umfassend:
- ein Kanülengehäuse (1) mit einer von diesem abstehenden flexiblen Kanüle (6), welche von einer sich im Kanülenkanal erstreckenden Führungsnadel (4) durchsetzt ist, wobei das Kanülengehäuse (1) einen Kanal (29) bildet, über welchen eine Flüssigkeit zur Eintrittsöffnung der Kanüle (6) geführt oder von dieser abgeführt werden kann, und wobei die Begrenzungswandungen des Kanals (29) in einem Bereich gegenüberliegend der Eintrittsöffnung der Kanüle (6) ein Dichtelement (10), insbesondere ein Septum (10) aufweisen, durch welches die Führungsnadel (4) aus dem Kanülengehäuse (1) austritt,
- ein Umlenkelement (30), welches gegenüberliegend dem Austrittsort der Führungsnadel (4) aus dem Kanülengehäuse (1) angeordnet ist und in welchem die Führungsnadel (4) axial verschieblich in einer Führung (21) umgelenkt ist, wobei die Führungsnadel (4) im Bereich zwischen der Kanüle (6) und ihrem Eintritt in das Umlenkelement (30) einen geradlinigen Verlauf aufweist und im Eintrittsbereich der Führung (21) des Umlenkelements (30) in Verlängerung dieses geradlinigen Verlaufes geführt ist, und
- eine Rückziehvorrichtung (2, 3, 5) für die Führungsnadel (4), mittels welcher die Führungsnadel (4) unter Umlenkung im Umlenkelement (30) soweit zurückgezogen werden kann, dass sie die Eintrittsöffnung der Kanüle (6) freigibt.

2. Anordnung nach Anspruch 1, **dadurch gekennzeichnet, dass** das Kanülengehäuse (1), das Umlenkelement (30) und die Rückziehvorrichtung (2, 3, 5) eine untrennbare Einheit bilden.

3. Anordnung nach Anspruch 1, **dadurch gekennzeichnet, dass** die Rückziehvorrichtung (2, 3, 5) derartig ausgebildet ist, dass die Führungsnadel (4) mit dieser in eine Rückzugendposition zurückgezogen werden kann, in welcher die Führungsnadel (4) vollständig aus dem Dichtelement (10) herausgezogen ist und die Spitze der Führungsnadel (4) innerhalb des Umlenkelements (30) oder der Rückziehvorrichtung (2, 3, 5) angeordnet ist, wobei das Kanülengehäuse (1), das Umlenkelement (30) und die Rückziehvorrichtung (2, 3, 5) derartig miteinander verbunden sind, dass diese bei nicht in der Rückzugendposition angeordneter Führungsnadel (4) eine untrennbare Einheit bilden und dass bei in der Rückzugendposition angeordneter Führungsnadel (4) die Rückziehvorrichtung (2, 3, 5) oder die Rückziehvorrichtung (2, 3, 5) und das Umlenkelement (30) vom Kanülengehäuse (1) entfernbar sind, wobei die Spitze der Führungsnadel (4) vor einem Zugriff geschützt innerhalb der entfernten Rückziehvorrichtung (2, 3, 5) bzw. des entfernten Umlenkelements (30) angeordnet ist.

4. Anordnung nach Anspruch 3, **dadurch gekennzeichnet, dass** die Rückziehvorrichtung (2, 3, 5) und das Umlenkelement (30) als untrennbare Einheit ausgebildet sind und die Spitze der Führungsnadel (4) in der Rückzugendposition innerhalb des Umlenkelements (30) angeordnet ist.

5. Anordnung nach einem der vorangehenden Ansprüche, **dadurch gekennzeichnet, dass** die Rückziehvorrichtung (2, 3, 5) zwei entlang von Führungen relativ zueinander bewegbare Bauteile (2, 3) aufweist, von denen ein erstes Bauteil (2) gegenüber dem Kanülengehäuse (1) feststehend ist, wobei das zweites Bauteil (3) gegenüber dem Kanülengehäuse (1) bewegbar ist und derartig mit der Führungsnadel (4) gekoppelt ist, dass bei einem Bewegen der beiden Bauteile (2, 3) relativ zueinander die Führungsnadel (4) vom zweiten Bauteil (3) zurückgezogen wird.

6. Anordnung nach Anspruch 5, **dadurch gekennzeichnet, dass** die beiden Bauteile (2, 3) als insbesondere translatorisch zusammenschiebbare Bauelemente (2, 3) ausgebildet sind.

7. Anordnung nach Anspruch 6, **dadurch gekennzeichnet, dass** die Zusammenschieberichtung der beiden Bauteile (2, 3) quer zur Richtung der Längserstreckung der Kanüle (6) liegt und/oder derartig orientiert ist, dass sie bei bestimmungsgemäss am Körper einer Person applizierter Anordnung im wesentlichen parallel zur Hautoberfläche im Bereich der Applikationsstelle verläuft.

8. Anordnung nach einem der Ansprüche 6 bis 7, **dadurch gekennzeichnet, dass** eines der beiden Bauteile (2, 3) zwei in einer Richtung quer zur Zusammenschieberichtung der Bauteile voneinander beabstandete Griffflächen (24, 25) zur Auflage der jeweiligen Fingerkuppen von Zeigefinger und Mittelfinger eines Benutzers aufweist und das andere der beiden Bauteile eine diesen beiden Griffflächen in Zusammenschieberichtung etwa mittig gegenüberliegende Grifffläche (13, 14) zur Auflage der Kuppe des Daumens des Benutzers aufweist, derart, dass das Zusammenschieben der beiden Bauteile (2, 3) durch eine der Betätigung einer Spritze ähnliche Fingerbewegung erfolgen kann.

9. Anordnung nach Anspruch 5, **dadurch gekennzeichnet, dass** die beiden Bauteile als gegeneinander verdrehbare Bauelemente ausgebildet sind.

10. Anordnung nach Anspruch 9, **dadurch gekennzeichnet, dass** die Verdrehachse der beiden Bauteile parallel zur Richtung der Längserstreckung der Kanüle (6) verläuft und/oder derartig orientiert ist, dass sie bei bestimmungsgemäss am Körper einer Person applizierter Anordnung im Wesentlichen senkrecht zur Hautoberfläche im Bereich der Applikationsstelle verläuft.

11. Anordnung nach einem der Ansprüche 5 bis 10, **dadurch gekennzeichnet, dass** die beiden Bauteile (2, 3) derartig ausgebildet sind, dass sie sich beim Zusammenschieben oder Gegeneinanderverdrehen, insbesondere in einer Bewegungsendposition, in welcher die Führungsnadel (4) maximal zurückgezogen ist, miteinander verrasten, derart, dass die Rückzugbewegung irreversibel ist.

12. Anordnung nach Anspruch 3 und nach einem der Ansprüche 5 bis 11, **dadurch gekennzeichnet, dass** das erste Bauteil (2) über einen Befestigungsmechanismus (28) entfernbar am Kanülengehäuse (1) befestigt ist, welcher durch das zweite Bauteil (3) verriegelt ist und erst nach einem Bewegen der beiden Bauteile (2, 3) relativ zueinander in eine Bewegungsendposition, in welcher die Führungsnadel in der Rückzugendposition angeordnet ist, freigegeben wird.

13. Anordnung nach Anspruch 12, **dadurch gekennzeichnet, dass** das Kanülengehäuse (1) einen Anschlussport (31) für eine Leitung (19) zum Zuführen einer Flüssigkeit in den Kanal (29) oder zum Abführen einer Flüssigkeit aus dem Kanal (29) aufweist, welcher erst nach einem Entfernen des ersten Bauteils (2) vom Kanülengehäuse (1) zugänglich ist.

14. Anordnung nach einem der vorangehenden Ansprüche, **dadurch gekennzeichnet, dass** die Aussenseite des Kanülengehäuses (1), auf welcher die Kanüle (6) von diesem absteht, im Wesentlichen eben ist, und insbesondere, dass die Kanüle (6) im Wesentlichen senkrecht von dieser Aussenseite absteht.

15. Anordnung nach einem der vorangehenden Ansprüche, dass die Aussenseite des Kanülengehäuses (1), auf welcher die Kanüle (6) von diesem absteht, eine insbesondere von einem Pflaster (7) gebildete Klebeschicht trägt, zur Befestigung des Kanülengehäuses (1) durch Kleben auf der Haut eines Patienten.

16. Anordnung nach einem der vorangehenden Ansprüche, **dadurch gekennzeichnet, dass** die Führungsnadel (4) im Umlenkelement (30) um 90° umgelenkt wird.

## Claims

1. Arrangement for particularly subcutaneously inserting a liquid into the body of a patient or for removing a liquid from the body of a patient, comprising:
- a cannula housing (1) with a flexible cannula (6) sticking out of it, which is penetrated by a guide needle (4) extending in the cannula channel, wherein the cannula housing (1) forms a channel (29) via which a liquid is guided to the inlet opening of the cannula (6) or from which it may be removed from it, and wherein the border wall of the channel (29) has a sealing element (10), particularly a septum (10), in the area opposite of the inlet opening of the cannula (6), through which the guide needle (4) exits the cannula housing (1),
- a deflector (30) which is arranged opposite of the exit location of the guide needle (4) from the cannula housing (1) and inside of which the guide needle (4) is deflected in a guidance (21) in an axially displaceable manner, wherein the guide needle (4) has a straight run in the area between the cannula (6) and its entry into the deflector (30) and is guided in the entry area of the guide (21) of the deflector (30) in extension to this straight run, and
- a retraction device (2, 3, 5) for the guide needle (4), by means of which the guide needle (4) may be retracted so far into the deflector during deflection, as to free the inlet opening of the cannula (6).

2. Arrangement according to claim 1, **characterized in that** the cannula housing (1), the deflector (30) and the retraction device (2, 3, 5) form an inseparable entity.

3. Arrangement according to claim 1, **characterized in that** the retraction device (2, 3, 5) is formed such that the guide needle (4) may be retracted with it in a retraction end position, in which the guide needle (4) is entirely pulled out of the sealing element (10) and the tip of the guide needle (4) is arranged within the deflector (30) or the retraction device (2, 3, 5), wherein the cannula housing (1), the deflector (30) and the retraction device (2, 3, 5) are connected in such a way that they form an inseparable entity when the guide needle (4) is not arranged in the retraction end position and **in that** the retraction device (2, 3, 5) or the retraction device (2, 3, 5) and the deflector (30) are removable from the cannula housing (1) when the guide needle (4) is arranged in the retraction end position, wherein the tip of the guide needle (4) is arranged inside of the remote retraction device (2, 3, 5) or the remote deflector (30) respectively, such that it is protected from access.

4. Arrangement according to claim 3, **characterized in that** the retraction device (2, 3, 5) and the deflector (30) are formed as inseparable entity and the tip of the guide needle (4) is arranged in the retraction end position inside of the deflector (30).

5. Arrangement according to one of the preceding claims, **characterized in that** the retraction device (2, 3, 5) has two components (2, 3) which are movable relatively to each other along guides, of which a first component (2) is firm with respect to the cannula housing (1), wherein the second component (3) is movable relatively to the cannula housing (1) and is coupled to the guide needle (4) is such a way that in case of a movement of both components (2, 3) relatively to each other the guide needle (4) is retracted from the second component (3).

6. Arrangement according to claim 5, **characterized in that** both components (2, 3) are formed as particularly translatory telescopic components (2, 3).

7. Arrangement according to claim 6, **characterized in that** the telescopic direction of both components (2, 3) lies transversally to the direction of the longitudinal elongation of the cannula (6) and/or is oriented in such a way that it runs substantially parallel to the skin surface in the application area, when the arrangement is applied as intended to a person's body.

8. Arrangement according to one of the claims 6 to 7, **characterized in that** one of both components (2, 3) has two mutually spaced gripping areas (24, 25) in a transversal direction with respect to the telescopic direction of the components, for placing the respective fingertips of forefinger and middle finger of a user and the other one of both components has a gripping area (13, 14) for placing the tip of the thumb of the user, lying approximately in the middle between said two gripping areas and opposite of them in telescopic direction, in such a way that the pushing together of both components (2, 3) may be done in a similar way to the finger movement used when operating a syringe.

9. Arrangement according to claim 5, **characterized in that** both components are formed as components which are rotatable relative to each other.

10. Arrangement according to claim 9, **characterized in that** the rotation axis of both components runs parallel to the direction of the longitudinal elongation of the cannula (6) and/or is oriented in such a way that it runs substantially perpendicular to the skin surface in the application area when the arrangement is applied as intended to a person's body.

11. Arrangement according to one of the claims 5 to 10, **characterized in that** both components (2, 3) are formed in such a way that they engage each other in a way that the retraction movement is irreversible in case of pushing them together or rotating them against each other, particularly in a movement end position in which the guide needle (4) is retracted to the maximum extent.

12. Arrangement according to claim 3 and to one of the claims 5 to 11, **characterized in that** the first component (2) is detachably attached to the cannula casing (1) by means of an attaching mechanism (28) which is locked by the second component (3) and is released into a movement end position in which the guide needle is arranged in the retraction end position only after a moving of both components (2, 3) relative to each other.

13. Arrangement according to claim 12, **characterized in that** the cannula housing (1) has an connecting port (31) for a conduct (19) for supplying a liquid into the channel (29) or for removing a liquid from the channel (29), which is accessible only after removing the first component (2) from the cannula housing (1).

14. Arrangement according to one of the preceding claims, **characterized in that** the outer side of the cannula housing (1), on which the cannula (6) is sticking out of the latter, is substantially even, and particularly **in that** the cannula (6) is sticking out substantially perpendicularly with respect to this outer side.

15. Arrangement according to one of the preceding claims, **characterized in that** the outer side of the cannula housing (1), on which the cannula (6) is sticking out of the latter, supports a glue layer, particularly formed by a plaster (7), for the attachment of the cannula housing (1) by gluing onto the skin of a patient.

16. Arrangement according to one of the preceding claims, **characterized in that** the guide needle (4) is deflected by 90° in the deflector (30).

## Revendications

1. Système pour l'introduction d'un liquide dans le corps d'un patient, en particulier par voie sous-cutanée, ou pour évacuer un liquide hors du corps d'un patient, comprenant :
- un boîtier de canule (1) avec une canule flexible (6) dépassant depuis celui-ci, laquelle est traversée par une aiguille de guidage (4) qui s'étend dans le canal de canule, le boîtier de canule (1) formant un canal (29) via lequel un liquide est guidé jusqu'à l'ouverture d'entrée de la canule (6) ou peut être évacué hors de celle-ci, et les parois de délimitation du canal (29) comportent, dans une région à l'opposé de l'ouverture d'entrée de la canule (6), un élément d'étanchéité (10), en particulier un septum (10), à travers lequel l'aiguille de guidage (4) sort hors du boîtier de canule (1),
- un élément de renvoi (30), lequel est agencé à l'opposé de l'emplacement de sortie de l'aiguille de guidage (4) hors du boîtier de canule (1) et dans lequel l'aiguille de guidage (4) est renvoyée, avec possibilité de translation axiale vers un guidage (21), de sorte que l'aiguille de guidage (4) présente, dans la région entre la canule (6) et son entrée dans l'élément de renvoi (30), un tracé rectiligne et est guidée, dans la région d'entrée du guidage (21) de l'élément de renvoi (30), dans le prolongement de ce tracé rectiligne, et
- un dispositif de rétraction (2, 3, 5) pour l'aiguille de guidage (4), au moyen duquel l'aiguille de guidage (4) peut être rétractée, avec renvoi dans l'élément de renvoi (30), aussi loin qu'elle libère l'ouverture d'entrée de la canule (6).

2. Système selon la revendication 1, **caractérisé en ce que** le boîtier de canule (1), l'élément de renvoi (30) et le dispositif de rétraction (2, 3, 5) forment une unité inséparable.

3. Système selon la revendication 1, **caractérisé en ce que** le dispositif de rétraction (2, 3, 5) est réalisé de telle façon que l'aiguille de guidage (4) peut être rétractée avec celui-ci dans une position de rétraction finale dans laquelle l'aiguille de guidage (4) est totalement extraite hors de l'élément d'étanchéité (10) et la pointe de l'aiguille de guidage (4) est agencée à l'intérieur de l'élément de renvoi (30) ou du dispositif de rétraction (2, 3, 5), le boîtier de canule (1), l'élément de renvoi (30) et le dispositif de rétraction (2, 3, 5) étant reliés les uns aux autres de telle façon que, lorsque l'aiguille de guidage (4) n'est pas agencée dans la position de rétraction finale, ceux-ci forment une unité inséparable, et lorsque l'aiguille de guidage (4) est agencée dans la position de rétraction finale, le dispositif de rétraction (2, 3, 5) ou bien le dispositif de rétraction (2, 3, 5) et l'élément de renvoi (30) peut/peuvent être enlevé(s) hors du boîtier de canule (1), et la pointe de l'aiguille de guidage (4) est agencée de façon protégée par rapport à un accès, à l'intérieur du dispositif de rétraction (2, 3, 5) enlevé ou de l'élément de renvoi (30) enlevé.

4. Système selon la revendication 3, **caractérisé en ce que** le dispositif de rétraction (2, 3, 5) et l'élément de renvoi (30) sont réalisés comme une unité inséparable, et dans la position de rétraction finale, la pointe de l'aiguille de guidage (4) est agencée à l'intérieur de l'élément de renvoi (30).

5. Système selon l'une des revendications précédentes, **caractérisé en ce que** le dispositif de rétraction (2, 3, 5) comprend deux composants structurels (2, 3) mobiles l'un par rapport à l'autre le long de guides, parmi lesquels un premier composant structurel (2) est stationnaire par rapport au boîtier de canule (1), tandis que le second composant structurel (3) est déplaçable par rapport au boîtier de canule et est couplé avec l'aiguille de guidage (4) de telle manière que lors d'un déplacement des deux composants structurels (2, 3) l'un par rapport à l'autre, l'aiguille de guidage (4) est rétractée par rapport au second composant structurel (3).

6. Système selon la revendication 5, **caractérisé en ce que** les deux composants structurels (2, 3) sont réalisés sous la forme d'éléments structurels (2, 3) capable d'être regroupés, en particulier par translation

7. Système selon la revendication 6, **caractérisé en ce que** la direction de regroupement des deux composants structurels (2, 3) est perpendiculaire à la direction de l'extension longitudinale de la canule (6) et/ou est orientée de telle façon que lorsque le système est appliqué conformément à sa destination sur le corps d'une personne, elle s'étend sensiblement parallèlement à la surface de la peau dans la zone de l'emplacement d'application.

8. Système selon l'une des revendications 6 à 7, **caractérisé en ce que** l'un des deux composants structurels (2, 3) comporte deux surfaces d'engagement (24, 25), écartées l'une de l'autre dans une direction transversale à la direction de regroupement des composants structurels, pour l'application des bouts de doigts respectifs de l'index et du majeur d'un utilisateur, et l'autre des deux composants structurels comporte une surface d'engagement (13, 14) opposée à ces deux surfaces d'engagement approximativement au milieu dans la direction de regroupement, pour l'application du bout du pouce de l'utilisateur, de telle façon que le regroupement des deux composants structurels (2, 3) peut avoir lieu par un mouvement des doigts semblable à l'actionnement d'une seringue.

9. Système selon la revendication 5, **caractérisé en ce que** les deux composants structurels sont réalisés sous forme d'éléments structurels capables de tourner l'un par rapport à l'autre.

10. Système selon la revendication 9, **caractérisé en ce que** l'axe de rotation des deux composants structurels est parallèle à la direction de l'extension longitudinale de la canule (6) et/ou orienté de telle façon que, lorsque le système est appliqué conformément à sa destination sur le corps d'une personne, il s'étend sensiblement perpendiculairement à la surface de la peau dans la région de l'emplacement d'application.

11. Système selon l'une des revendications 5 à 10, **caractérisé en ce que** les deux composants structurels (2, 3) sont réalisés de telle façon que, lors du regroupement ou de la rotation relative, en particulier dans une position de fin de mouvement dans laquelle l'aiguille de guidage (4) est rétractée au maximum, ils s'enclenchent l'un avec l'autre de telle façon que le mouvement de rétraction est irréversible.

12. Système selon la revendication 3 et selon l'une des revendications 5 à 11, **caractérisé en ce que** le premier composant structurel (2) est fixé de façon amovible sur le boîtier de canule (1) via un mécanisme de fixation (28) qui est verrouillé par le second composant structurel (3) et qui n'est libéré qu'après un déplacement des deux composants structurels (2, 3) l'un par rapport à l'autre jusque dans une position de fin de déplacement dans laquelle l'aiguille de guidage est agencée dans la position de rétraction finale.

13. Système selon la revendication 12, **caractérisé en ce que** le boîtier de canule (1) comporte un orifice de raccordement (31) pour un conduit (19) destiné à l'introduction d'un liquide dans le canal (29) ou à l'évacuation d'un liquide hors du canal (29), qui n'est accessible qu'après avoir enlevé le premier composant structurel (2) depuis le boîtier de canule (1).

14. Système selon l'une des revendications précédentes, **caractérisé en ce que** le côté extérieur du boîtier de canule (1), sur lequel la canule (6) dépasse du boîtier, est sensiblement plan, et en particulier **en ce que** la canule (6) dépasse sensiblement perpendiculairement depuis ce côté extérieur.

15. Système selon l'une des revendications précédentes, **caractérisé en ce que** le côté extérieur du boîtier de canule (1), sur lequel la canule (6) dépasse du boîtier, porte une couche de colle, formée en particulier par un pansement (7), pour la fixation du boîtier de canule (1) par collage sur la peau d'un patient.

16. Système selon l'une des revendications précédentes, **caractérisé en ce que** l'aiguille de guidage (4) est renvoyé de 90° dans l'élément de renvoi (30).
